# EUROPÄISCHE PATENTSCHRIFT

(11) **EP 0 526 682 B1**
(45) Veröffentlichungstag und Bekanntmachung des Hinweises auf die Patenterteilung: **05.03.1997**
(21) Anmeldenummer: 91810618.8
(22) Anmeldetag: 07.08.1991
(51) Int. Cl.: A61F 2/30, A61F 2/28, B22F 7/00, B21F 43/00, A61L 27/00, A61F 2/44, A61F 2/32, A61F 2/38, A61C 8/00

(54) **Endoprothese mit einem Metalldrahtwerk**
Endoprosthesis with a metal wire mesh
Endoprothèse avec un tissu en fils métalliques

(43) Veröffentlichungstag der Anmeldung: 10.02.1993
(73) Patentinhaber: OSCOBAL AG, 2545 Selzach (CH)
(72) Erfinder: Mittelmeier, Heinz, Prof. Dr. med., W-6650 Homburg-Schwarzenbach (DE); Leu, Beat, CH-6052 Hergiswil (CH)
(74) Vertreter: Seehof, Michel

(56) Entgegenhaltungen:
- EP-A- 0 224 890
- EP-A- 0 278 205
- EP-A- 0 338 976
- WO-A-90/08520
- CH-A- 368 309
- FR-A- 2 115 576
- FR-A- 2 331 320
- GB-A- 2 184 458
- US-A- 4 397 359
- US-A- 4 997 445
- Lueger Lexikon der Technik,Band 9, Seiten 46 und 47

## Beschreibung

Die vorliegende Erfindung bezieht sich auf eine Endoprothese mit einem Metalldrahtwerk aus elastischen Metalldräten für die Endoprothetik sowie auf ein Verfahren zur Herstellung dieser Prothese Die Endoprothetik des Skelettes bezog sich in erster Linie auf den Ersatz von zerstörten Gelenken, wird aber zunehmend auch zum Ersatz von anderweitigen Skelettabschnitten an Schäften der Gliedmassen, Knochen, am Becken sowie zum Ersatz von Wirbelkörpern, Schädelkalotten und zur Zahn- bzw. Gebissverankerung herangezogen. Als eines der wichtigsten Probleme stellte sich dabei die Verankerung der Prothesen im Knochen heraus.

In letzter Zeit, als erkannt wurde, dass die Verankerung der Prothesen mittels Knochenzement zu erheblichen Problemen führt, konzentrierten sich die Anstrengungen hauptsächlich darauf, Prothesen mit Oberflächen zu versehen, an denen das Knochengewebe dauerhaft anwachsen kann. Dabei spielen verschiedene Faktoren eine Rolle wie zum Beispiel die Porosität, Materialbeschaffenheit und Verbund mit der Prothese. Eine der Entwicklungen geht dahin, Geflechte aus Metalldrähten zu verwenden, wobei stellvertretend für eine Reihe von Patenten und Patentanmeldungen die US-A-4 976 738 genannt sei, die eine metallische Beschichtung aus mehreren Schichten von miteinander verwobenen Metalldraht-Geweben, vorzugsweise Titandrähten, beschreibt, wobei die Maschengrösse von aussen nach innen zur Prothese hin abnimmt. Eine solche Beschichtung wird zwar für Kunststoffprothesen beschrieben, ist jedoch auch für Metallprothesen verwendbar. Während hier versucht wird, das Problem der geeigneten Porengrösse zu lösen, lässt die Elastizität zu wünschen übrig. Nach dem Aufsintern auf eine Prothesenoberfläche wird das Netz relativ steif und seine Metallstrukturen vermögen den elastischen Bewegungen des einwachsenden und aufliegenden Knochens nicht ausreichend zu folgen. Deshalb ergeben sich bei der Aufschichtung solcher Strukturen auf Metallprothesen Probleme, weil sie keinen strukturellen Elastizitätsübergang zwischen dem relativ steifen Metall und dem Knochengewebe oder gar der Spongiosa erlauben, so dass es infolge von Scherbewegungen in der Aussenzone teilweise nur zu bindegewebigen statt knochigen Verbindungen kommt.

Dies trifft auch auf die WO 90/08520 zu, in welcher ein Drahtgewebe offenbart wird, das mit Glaskeramik beschichtet wird. Dadurch entsteht ein relativ steifes Gebilde.

Bei der Verwendung einer aus dünnen, wellenförmigen Blechen bestehenden Auflage auf Metallimplantate wurde zwar versucht, das Problem der Elastizität zu lösen, doch ergeben sich dort andere Probleme, besonders bezüglich der Porengrösse sowie ein relativ aufwendiges Herstellungsverfahren. Diese Knochenimplantatauflage wird in der US-A-4 969 907 beschrieben.

Aus der US-A-4 997 445 ist eine Endoprothese gemäss Oberbegriff von Patentanspruch 1 bekannt, bei der auf der mit dem Knochen in Kontakt kommenden Unterseite ein unwillkürlich angeordnetes, komprimiertes Metallgeflecht aus elastischen Metalldrähten auf einem Metallschirm aufgebracht ist, das zwar Poren aufweist aber wenig elastisch ist.

Ausserdem ist aus der FR-A-2 331 320 eine Prothese bekannt, die teilweise mit einer Spiralfederschicht versehen ist, um dort ein besseres Anwachsen des Knochens zu gewährleisten. Dazu müssen die Spiralfedern teilweise in der Oberfläche der Prothese eingelassen werden und beim Aufbringen haben sie noch keinen festen Halt.

Es ist von diesem vorbekannten Stand der Technik ausgehend Aufgabe der vorliegenden Erfindung eine Endoprothese mit einem Metalldrahtwerk aus elastischen Metalldrähten anzugeben, dessen Porenstruktur das gute Hineinwachsen des Knochengewebes ermöglicht und eine relativ hohe Bewegungselastizität erreicht, wodurch eine dauernde Verankerung der Prothese gewährleistet wird, und ein Verfahren, das eine einfache Herstellung solcher Prothesen erlaubt, wobei die Endoprothese eine Metall- oder Kunststoffendoprothese sein kann.

Diese Aufgaben werden mit einer im unabhängigen Anspruch 1 definierten Endoprothese und mit einem in Anspruch 6 angegebenen Verfahren gelöst. Dabei hat das Metalldrahtwerk die Form eines Hohlmaschengestrickes eines Rete milanese. Solche Strukturen sind in der Schmuckindustrie beispielsweise aus der FR-A-2 115 576 oder aus der CH-A-368 309 an sich für Uhrenarmbänder und dergl. bekannt.

Die Erfindung wird im folgenden anhand einer Zeichnung von Ausführungsbeispielen näher erläutert.
Figur 1 zeigt, in Draufsicht, schematisch ein einschichtiges Drahtgestrick gemäss der Erfindung,
Figur 2 zeigt einen Schnitt von Figur 1,
Figur 3 zeigt, im Schnitt, eine mit dem Gestrick gemäss Figur 1 beschichtete Endoprothese,
Figur 3a zeigt einen Schnitt gemäss der Linie IIIa-IIIa von Figur 3,
Figur 4 zeigt im vergrösserten Massstab einen Schnitt gemäss der Linie IV-IV in Figur 3a,
Figur 5 zeigt die Verwendung des Gestricks gemäss Figur 1 an einem Knochenschaft-Zwischenstück am Oberschenkelknochen,
Figur 6 zeigt die Verwendung eines Gestricks an einem Knochenschaft-Zwischenstück am Oberarm,
Figur 7 zeigt einen Schnitt gemäss der Linie VII-VII in Figur 6,
Figur 8 zeigt einen Schnitt gemäss der Linie VIII-VIII in Figur 6,
Figur 9 zeigt die Verwendung eines erfindungsgemässen Gestricks an einer Schienbeinkopf-Prothese aus Kunststoff,
Figur 10 zeigt die Verwendung eines erfindungsgemässen Gestricks an einer Schienbeinkopf-Prothese aus Kunststoff auf Metallunterlagsscheibe,
Figur 12 zeigt in einem Schnitt die Verwendung eines erfindungsgemässen Gestricks an einer Hüftpfannenprothese,

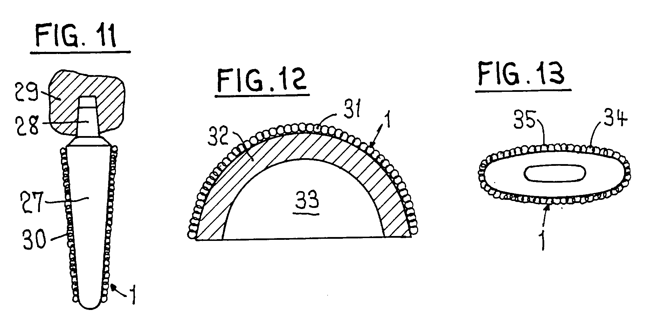

Figur 11 zeigt in einem Schnitt die Verwendung eines erfindungsgemässen Gestricks an einer Zahnwurzel,
Figur 13 zeigt die Verwendung eines erfindungsgemässen Gestricks bei einer künstlichen Bandscheibe,

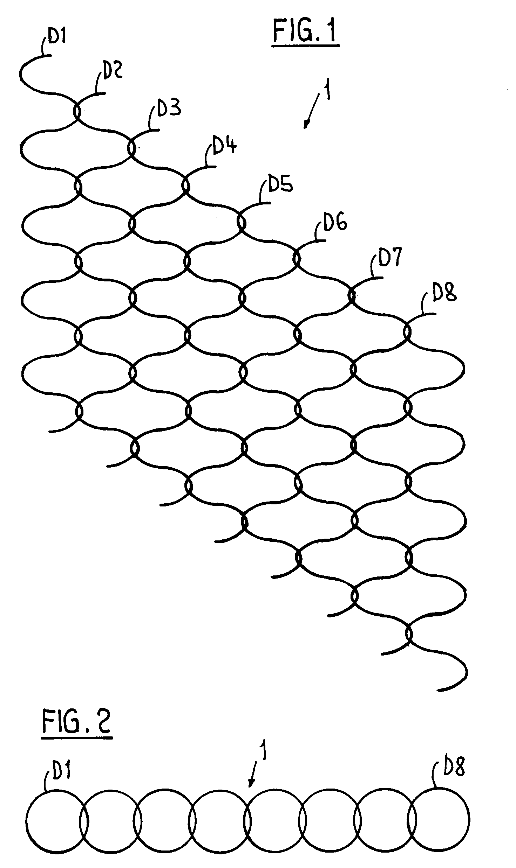

Als Ausgangsmaterial zur Herstellung des Drahtgestricks gemäss den Figuren 1 und 2 sind körperverträgliche Metalldrähte zu verwenden, wobei sich besonders solche mit hoher Duktilität eignen, zum Beispiel aus Titan, rostfreiem Stahl oder anderen Edelstählen, einer Kobalt-Chrom-, Kobalt-Molybdän-, Titan- oder Goldlegierungen. Solche Drähte sind im Handel erhältlich. In der Regel werden Einfachdrähte verwendet, doch finden auch geflochtene Drähte für spezielle Anwendungen Verwendung. Die Drahtstärke ist der Grösse des Verankerungskörpers, respektive Implantates angepasst und liegt zwischen 0,030 und 1,0 mm, vorzugsweise in der Grössenordnung von 0,1 bis 0,5 mm, während die lichte Porengrösse je nach Verwendungszweck bei einer Grösse von 0,2 - 1,5 mm liegt, siehe Ausführungsbeispiele I-II.

Die Herstellung des einschichtigen Drahtgestricks gemäss den Figuren 1 und 2 erfolgt mit an sich bekannten Drahtstrickmaschinen, wie sie beispielsweise in der Schmuckindustrie gebräuchlich sind. Dort werden solche Drahtstrickmaschinen zur Herstellung von flexiblen Halsketten oder Armbändern verwendet. Dabei ist vorzugsweise daran gedacht, das Gestrick in Form verschlungener und verbundener Spiralfedern auszuführen. Die Spiralfeder beinhaltet zunächst einmal die Möglichkeit der Gestaltung von Spiralschlingen verschiedenen Durchmessers und verschiedener Steigung und damit Variabilität der Porosität. Ausserdem beinhaltet sie in besonderer Weise das gewünschte elastische Federprinzip, welches im noch ungesinterten Zustand eine besonders gute Anformbarkeit an verschieden gestaltete Prothesenoberflächen oder als Implantat ermöglicht, aber auch im gesinterten Zustand eine relativ hohe elastische Beweglichkeit seiner Maschen gewährleistet, so dass dieselben die elastischen Verformungen des Knochens im Sinne der weiter oben angeführten Zielsetzung gut mitmachen können.

Ausserdem ist der Spiralfederverbund relativ leicht herstellbar. Das Prinzip des elastischen Federverbundes ist in der Schmuckindustrie als sogenanntes "rete milanaise" bekannt. In der Textilindustrie sind solche Hohlmaschengestricke auch als Ketten-oder Maschenware bekannt. In der Schmuckindustrie wird jedoch dieses Gestrick in der Regel in flexibler, unversinterter Art, meistens auch noch flächenartig verpresst verwendet. Im Unterschied dazu dient das erfindungsgemässe Gestrick lediglich als gut anformbares Zwischenprodukt, welches erst durch die nachfolgende Sinterung die gewünschte Formstabilität bei relativ hoher verbleibender Elastizität erhält, das im verpressten Zustand die gewünschte Porenstruktur verlieren würde. Würde man einen Maschenwarenstrumpf, beispielsweise gemäss US-A-4 064 567, ohne Versinterung auf Prothesenschäfte aufziehen, käme es schon beim Einführen der Prothese zum Zusammenstauchen der Maschenware und bei der Belastung zu ständigen Scheuerreibungen mit erheblichem Metallabrieb und nachfolgender Fremdkörperentzündung des Gewebes im Sinne der sogenannten Metallose.

Die Metall-Hohlmaschengestricke können, wie jede andere Maschenware auch, in den unterschiedlichsten Teilen und Formen gestrickt werden, ökonomisch jedoch am vorteilhaftesten als sogenannte Endlosware und können dann auf die gewünschte Grösse und Auflageform, beispielsweise in Form kurzer Schlauchabschnitte zum Aufziehen auf Prothesenstiele oder aber ovale Teile zum Aufsintern auf scheibenartige Prothesen-Auflageflächen, beispielsweise Schienbein-Plateaus der Knieprothesen zugeschnitten werden.

Nach dem Aufziehen bzw. Auflegen auf metallische Verankerungsflächen der Prothesen werden die derart noch locker beschichteten Prothesen in einen Sinterofen eingebracht, der unter Berücksichtigung der Metallart gewöhnlich unter Vakuum steht oder eine Edelgasatmosphäre aufweist. Im Sinterofen werden bei Niedertemperatursinterung (Diffusionshaftung), beispielsweise bei Temperaturen von 6oo-700 C° das Metalldrahtgestrick auf die Auflagefläche aufgesintert und die Maschenkontaktflächen gegeneinander versintert. Anstatt das Gestrick auf die Metallprothese aufzusintern ist es auch möglich, dieses vorzusintern und anschliessend durch Nieten, Schweissen, Anschrauben oder Anklemmen an der Prothese zu befestigen.

Beim Beschichten der Prothesen aus einem thermoplastischen Kunststoff, beispielsweise Polyäthylen, werden die flächenhaften, haubenartigen oder strumpfartigen Gestricke im temperierten Zustand auf die durch Erwärmung erweichten Beschichtungsflächen des Kunststoffes aufgelegt und soweit eingepresst, dass die Schlingen des Gestricks nur teilweise in den Kunststoff eingeschmolzen werden, teilweise aber noch daraus hervorstehen, damit in den freien Bereich das Einwachsen des Knochens und damit die "Einwachsfixierung" der Prothese erfolgen kann.

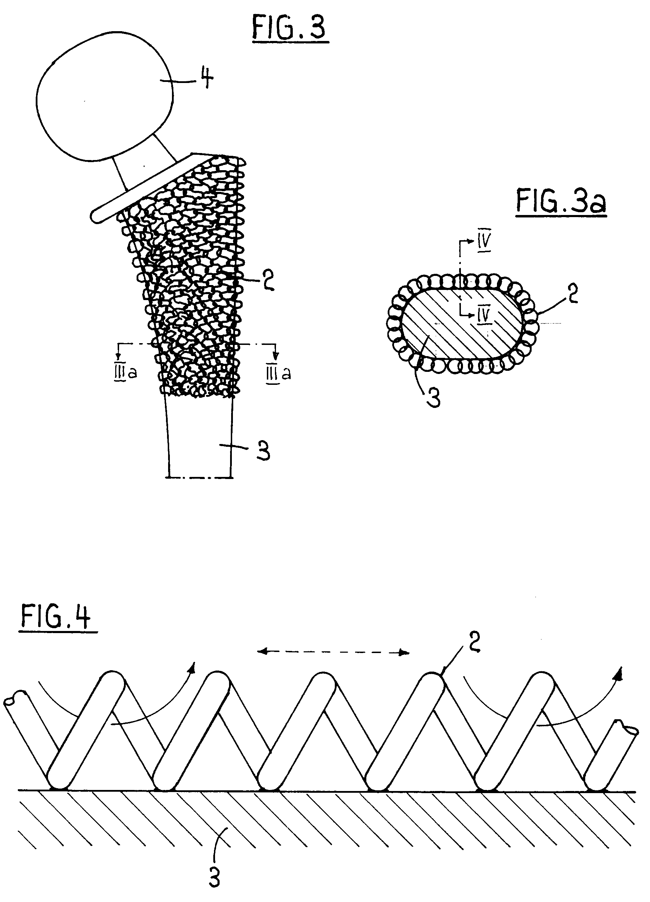

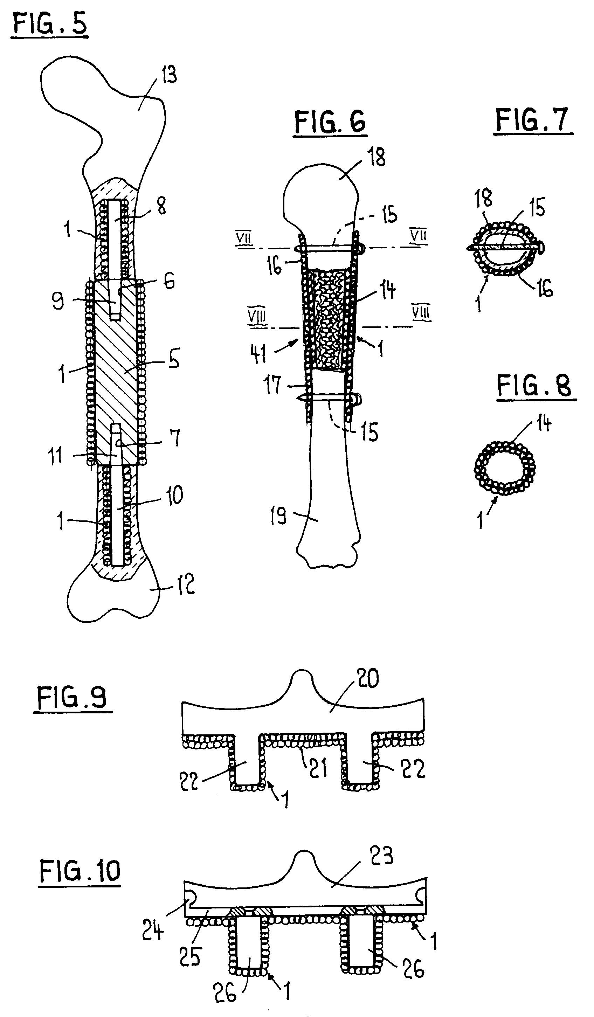

In Figur 1 ist eine mögliche Ausführungsform eines Gestricks dargestellt und man erkennt die spiralförmigen Drähte D1, 3, 5, 7 usw., die mit den Drähten D2, 4, 6, 8 usw. verschlungen sind, siehe auch Figur 3a. Dieses Gestrick entspricht dem eingangs erwähnten Typ "rete milanaise".

Für besondere Zwecke kann es vorteilhaft sein, mehrschichtige Gestricke zu verwenden, entweder derart, dass die Gestricke übereinandergelegt und nur versintert (siehe Beispiele gemäss den Figuren 14-16) oder aber derart, dass die Gestricke von Anfang an mehrschichtig hergestellt werden. Dies wäre besonders bei der Verwendung des "rete milanaise" durchführbar.

Das Aufstecken von zylindrischen Strümpfen 2 auf konische Prothesenschäfte 3 (siehe Figur 3) führt dazu, dass das Gestrick im breiteren Teil gestreckte, d.h. gedehnte weitere Maschenräume aufweist als im schmäleren Teil, wo die Maschen dann in mehr zusammengestauchter Form vorliegen und folglich kleinere Poren bilden. Dies ist jedoch kein Nachteil, da die stärkere Befestigung in grösseren Poren mit entsprechend kräftigeren Knochenzapfen im gelenknahen Bereich der Knochen (Epi-Methaphyse) und dagegen eine etwas schwächere Verzahnung im mittleren Schaftbereich des Knochens (Diaphyse) angestrebt wird, wobei dies nicht nur für Hüftgelenkprothesen gilt. In diesem Falle wäre es ausserdem möglich, von vorne herein konische Gestricke mit zu bzw. umgekehrt abnehmender Maschenzahl herzustellen, wie dies beim Textilstricken üblich ist.

Selbstverständlich können auch andere Formen von Gestricke, abweichend von den vorhergehend beschriebenen Endlosgestricke oder konischen Gestricke hergestellt werden, die unter Umständen eine bessere Anpassung an die Prothesenform oder an das gewünschte Knochenersatzteil ermöglichen, z. B. in Form von Hauben, Säckchen, zylindrischen Rollen usw.

Das erfindungsgemässe Hohlmaschengestrick kann für alle Arten Prothesen wie Schulter-, Ellbogen-, Finger-, Hüftgelenk-, Knie-, Springgelenk- und Zehengelenkprothesen verwendet werden und ebenso für den Ersatz von Schaftknochen und als Knochenersatz bei Wirbeln, usw.

Neben den bereits erwähnten Drahtmaterialien ist es auch möglich, Drähte mit osteotrop-bioaktiven Eigenschaften zu verwenden oder bereits gesinterte Gestricke nachträglich in entsprechender Weise mit Kalziumsalözen, insbesondere auch Hydroxylapatit zu beschichten, wobei zum Beispiel das Plasmaspray-Verfahren verwendet werden kann. Hierdurch kann das Heranwachsen des Knochens an die derart bioaktivierten Metalloberflächen des Prothesenstiels und vor allem auch der Gestrick-Beschichtung beschleunigt werden.

### Ausführungsbeispiele

### 1. Hüftgelenk-Prothese gemäss Figur 3.

Ein Gestrick 1 vom Typ "rete milanaise" aus 0,3 mm Titandraht mit einem Maschendurchmesser von 2 mm, somit zentraler Porengrösse von 1,2 mm in Schlauchform mit einem Schlauchdurchmesser von 20 mm wird auf einen konischen Hüftprothesenstiel 3 aus hochfester Titan-Legierung von etwa 180 mm Länge im proximalen ersten Drittel, also auf etwa 60 mm Länge, so aufgesteckt, dass das Gestrick im oberen Bereich stark gestreckt fest auflegt, im unteren Bereich dagegen im ungedehnten Zustand. Gegebenenfalls kann am unteren Ende noch ein zirkulärer Titandrahtfaden zur Befestigung verwendet werden oder der Schlauch kann spiralig aufgewickelt werden, um die Spannung zu erhöhen.

Anschliessend wird das Titan-Drahtgeflecht in einem Sinterofen entweder im Vakuum oder in Argonatmosphäre auf den Stiel aufgesintert und an den Maschenkontaktstellen versintert. Auch wird das Prothesenteil nach weiterer Bearbeitung wie Konusfräsung zum Aufstecken eines metallischen oder keramischen Hüftkopfes 4 und Reinigung zur Verpackung, Sterilisation und Implantation bereitgestellt.

In Figur 4 ist schematisch dargestellt, wie die weiten Maschenräume das Einwachsen des Knochengewebes K, durch die Pfeile symbolisiert, begünstigt und die relativ grosse elastische Beweglichkeit (gestrichelter Pfeil) auch nach dem Versintern erhalten bleibt.

### 2. Knochenschaft-Zwischenstück am Oberschenkelknochen gemäss Figur 5.

Das zylindrische Zwischenstück 5 weist an beiden Enden je eine konische Bohrung 6 bzw. 7 auf, in die Verankerungsstiele eingesetzt sind. Der proximale Verankerungsstiel 8 weist einen der konischen Bohrung entsprechenden Konus 9 auf und wird in variabler Länge hergestellt, während der distale Verankerungsstiel 10 einen entsprechenden Konus 11 aufweist. Dabei kann auch das Zwischenstück eine angepasste, unterschiedliche Länge haben. Sowohl die Verankerungsstiele als auch das Zwischenstück 5 werden mit einem erfindungsgemässen Gestrick 1 überzogen und anschliessend wie im vorhergehenden Ausführungsbeispiel gesintert. Vorgängig können die Gestricke mit Einzeldrahtschlaufen befestigt werden.

### 3. Knochenschaft-Zwischenstück am Oberarm gemäss Figur 6.

Das Knochenschaft-Zwischenstück 41 besteht aus einem doppelschichtigen Gestrick 14, das mittels Schrauben 15 an einem Stumpf 16, bzw. 17 der Knochenabschnitte 18 bzw. 19 befestigt werden kann und anschliessend wie in den vorhergehenden Beispielen gesintert wird.

### 4. Schienbeinkopfprothese gemäss Figur 9.

Die an sich bekannte Schienbeinkopfprothese 20 aus Kunststoff wird an ihrer Unterseite mit einem doppelschichtigen Gestrick 21 versehen, während die Verankerungszapfen 22 mit dem einschichtigen Gestrick 1 beschichtet werden, worauf die Beschichtungen unter Erwärmung teilweise eingepresst werden.

### 5. Schienbeinkopfprothese auf Metallunterlagsscheibe gemäss Figur 10.

Die Schienbeinkopfprothese 23 aus Kunststoff ist über einen Schnappverschluss 24 mit einer Metallunterlagsscheibe 25 verbunden. In diese Metallunterlagsscheibe 25 sind Verankerungszapfen 26 eingeschraubt. Auch hier sind sowohl die Unterseite der Prothese als auch die Zapfen mit einem Gestrick 1 versehen, wobei das Gestrick in einem Sinterofen wie vorgängig beschrieben, aufgesintert wird. Dabei ist es zweckmässig, das Drahtgestrick mittels abnehmbaren Klemmen auf der unteren Plateauseite anzupressen. Nach der Sinterung können dann die Pressklemmen abgenommen werden, das Prothesenstück wird gereinigt und der weiteren Bearbeitung zugeführt, anschliessend strahlensterilisiert und verpackt.

### 6. Zahnwurzelprothese gemäss Figur 11.

Der Zahnwurzelprothese 27, beispielsweise mit einer Länge von 20 mm und konisch sich verjüngend, mit einem oberen Durchmesser von 4 mm, weist einen Aufsteckkonus 28 zur Aufnahme eines Aufbaus auf, beispielsweise eine Krone 29, oder ein Gebiss, und wird mit einem feinen schlauchartigen Drahtgestrick 30 mit einem Drahtdurchmesser von 0,1 mm und einer Porengrösse von 0,25 mm bestückt und einem Sinterofen zugeführt, in welchem das Gestrick mit dem Anker versintert wird. Danach erfolgt die übliche Reinigung, Nachbearbeitung, Sterilisation und Verpackung.

### 7. Hüftgelenkpfanne gemäss Figur 12.

Zuerst wird ein hemisphärisches kappenförmiges Drahtgestrick 31 mit einem Durchmesser von beispielsweise 50 mm hergestellt und auf eine Keramikkugel des gleichen Durchmessers aufgezogen und einer Kontaktsinterung der Maschenberührungspunkte unterzogen. Daraus ergibt sich eine in sich versteifte, dennoch elastische Drahtgestrickhemisphäre. Diese wird nun auf eine hemisphärische Hüftgelenkpfanne 32 aus Kunststoff mit dem gleichen Aussendurchmesser von 50 mm aufgesetzt und unter Erwärmung auf die Fliesstemperatur des Kunststoffes darauf aufgepresst, so dass die metallischen Schlingen des Gestricks teilweise in die erweichte Kunststoffoberfläche eindringen und sich dort fest verzahnen können. Nach dem Abkühlen ist der Kunststoff mit dem halbkugligen metallischen gesinterten Drahtgestrick fest verbunden. Danach erfolgt mittels einer Nachbearbeitung mit spanabhebendem Verfahren oder thermischen Oberflächen-Nachpressung die Schaffung der eigentlichen Pfannenhöhle 33 zur Aufnahme des Hüftgelenkkopfes entsprechend einem üblichen Hüftgelenkkopfdurchmesser von beispielsweise 28 oder 32 mm. Anschliessend erfolgt die Strahlensterilisation und Verpackung.

Selbstverständlich kann es sich dabei auch um eine Hüftgelenkpfanne anderer Aussenform, beispielsweise einer konischen Form, handeln mit entsprechendem vorgesintertem Gestrick. Bei diesem wie den vorhergehenden Beispielen wird in der Regel als Kunststoff Polyäthylen verwendet, doch kann selbstverständlich auch ein anderes dazu geeignetes Material verwendet werden.

### 8. Künstliche Bandscheibe gemäss Figur 13.

Zunächst wird im spanabhebenden oder Pressverfahren ein scheibenförmiges Kunststoffstück 34 von beispielsweise 30 mm Durchmesser hergestellt. Es werden gleich grosse Ausschnitte aus einem Endlos-Titan-Drahtgestrick 35 nach deren Sinterung und Verfestigung an der Ober- und Unterseite auf das Polyäthylenstück flächig aufgelegt und in einem Temperierofen gebracht, dessen Temperatur bis zur Schmelzgrenze des Kunststoffes hochgefahren wird. Nach Anschmelzen der Ober- und Unterseite des Kunststoffes werden die beiden Drahtgeflechtscheiben mechanisch oder hydraulisch in die Polyäthlyenoberfläche eingepresst, so dass der Kunststoff die anliegenden Teile der Gestrickmaschen umfliesst und das Gestrick somit am Kunststoff nach dem Erkalten festhaftet, im übrigen aber noch teilweise für das Einwachsen des Knochens freibleibt.

Zur Herstellung besserer elastischer Dämpfung kann auch eine leicht kugelig gewölbte Kunststoffscheibe hergestellt und zentral ausgehöhlt sowie schliesslich an den leicht gewölbten Oberflächen mit einem entsprechend geformten vorgesinterten Drahtgestrick in gleicher Weise versehen werden.

Es ist aber auch möglich, den Kunststoffkern in ein taschenförmiges Gestrick passender Grösse einzuführen und danach die thermische Pressverbindung zwischen dem Drahtgestrick und dem Kunststoff herzustellen. Zur Herstellung der künstlichen Bandscheibe wird vorteilhafterweise Polyäthylen verwendet.

## Patentansprüche

1. Endoprothese mit einem Metalldrahtwerk aus elastischen Metalldrähten, dadurch gekennzeichnet, dass das Metalldrahtwerk aus einem gesinterten Hohlmaschengestrick nach Art eines "Rete milanese" besteht, wobei die Endoprothese entweder eine Metall-Endoprothese (3, 5, 27) ist und das Hohlmaschengestrick (1, 14, 30) auf die Endoprothese aufgesintert ist oder die Endoprothese eine Kunststoff-Endoprothese (20, 23, 32, 34) ist und das versinterte Hohlmaschengestrick (1, 21, 31, 35) teilweise in der Oberfläche der Prothese eingeschmolzen ist.

2. Endoprothese nach Anspruch 1, dadurch gekennzeichnet, dass die Drähte des Metalldrahtwerkes aus Titan, rostfreiem Stahl, einer Kobalt-Chrom-, Kobalt-Molybdän-, Titan- oder Goldlegierung bestehen.

3. Endoprothese nach Anspruch 1 oder 2, dadurch gekennzeichnet, dass die Maschen des Metalldrahtwerkes einen Porendurchmesser von 0,2 - 1,5 mm und die Drähte einen Durchmesser von 0,03 - 1,0 mm aufweisen.

4. Endoprothese nach einem der Ansprüche 1 bis 3, dadurch gekennzeichnet, dass das Metalldrahtwerk entweder von sich aus osteotrop-bioaktive Eigenschaften aufweist oder mit Kalziumsalzen, insbesondere Hydroxylapatit, oder mit Keramik beschichtet ist.

5. Endoprothese nach Anspruch 1 als künstliche Bandscheibe, mit einem versinterten Metalldrahtwerk (35) um eine Kunststoffscheibe (34).

6. Endoprothese nach einem der Ansprüche 1 bis 5, dadurch gekennzeichnet, dass das Metalldrahtwerk Maschen unterschiedlicher Porengrösse aufweist.

7. Verfahren zur Herstellung einer Endoprothese nach Anspruch 1, dadurch gekennzeichnet, dass Metalldrähte auf einer Drahtstrickmaschine, die ausgebildet ist, ein Gestrick des Typs "Rete milanese" herzustellen, zu einem Hohlmaschengestrick geformt werden und das Hohlmaschengestrick anschliessend entweder auf die Metall-Endoprothese aufgebracht und mit deren Oberfläche versintert wird oder in die endgültige Form gebracht und in einem Ofen im Vakuum oder mit nichtoxydierender Atmosphäre, vorzugsweise mit einer Argonatmosphäre, gesintert wird, wobei eine niedere Temperatur in einem Bereich von etwa 600 - 700 °C verwendet wird und anschliessend unter Erwärmung in die Oberfläche der Kunststoff-Endoprothese teilweise eingepresst wird.

8. Verfahren nach Anspruch 7, dadurch gekennzeichnet, dass das Hohlmaschengestrick ein- oder mehrschichtig, als Stück- oder Endlosware oder der vorgesehenen Form angepasst hergestellt wird.

9. Verfahren nach Anspruch 7, dadurch gekennzeichnet, dass das gesinterte Drahtwerk aus einem osteotrop-bioaktiven Material besteht oder mit einem solchen Material, insbesondere Hydroxylapatit, beschichtet wird.

10. Verfahren nach einem der Ansprüche 7 bis 9 zur Herstellung einer Beschichtung eines Schaftes einer Hüftgelenkprothese, dadurch gekennzeichnet, dass das Gestrick aus Titan-Drähten in Form eines zylindrischen Strumpfes über den oberen, trapezförmigen Teil des metallischen Schaftes aufgezogen und in einem Sinterofen aufgesintert wird, wobei im oberen Teil gröbere und im unteren Teil feinere Maschenporen entstehen.

11. Verfahren nach einem der Ansprüche 7 bis 9 zur Herstellung einer Beschichtung einer Hüftgelenkpfanne aus Kunststoff, dadurch gekennzeichnet, dass ein kappenförmiges Gestrick auf eine Keramikform gleicher Dimension wie die Hüftgelenkpfanne aufgezogen und versintert wird und das derart versteifte Gestrick auf die Hüftgelenkpfanne aus Kunststoff aufgesetzt und bei der Fliesstemperatur des Kunststoffes derart eingepresst wird, dass die Schlingen des Gestricks nur teilweise in die Oberfläche der Hüftgelenkpfanne eindringen können, woraufhin die Nachbearbeitung erfolgt.

12. Verfahren nach einem der Ansprüche 7 bis 9 zur Herstellung einer künstlichen Bandscheibe, dadurch gekennzeichnet, dass ein scheibenförmiges Kunststoffstück von beispielsweise 30 mm Durchmesser hergestellt wird, zwei Ausschnitte gleichen Durchmessers aus einem Endlos-Titan-Drahtgestrick nach deren Versinterung an beiden Seiten der Scheibe aufgelegt und in einem Ofen bei der Fliesstemperatur des Kunststoffes teilweise eingepresst werden.

## Claims

1. Endoprosthesis comprising a metal wire structure formed by elastic metal wires, characterised by the fact that the metal wire structure consists of a sintered, hollow mesh knitting of the "rete milanese" kind, the endoprosthesis being a metal endoprosthesis (3, 5, 27) where the hollow mesh knitting (1, 14, 30) is sintered upon the endoprosthesis, or the endoprosthesis is a synthetic material endoprosthesis (20, 23, 32, 34) and the sintered hollow mesh knitting (1, 21, 31, 35) is partially melted into the surface of the prosthesis.

2. Endoprosthesis according to claim 1, characterised by the fact that the wires of the metal wire structure are made of titanium, stainless steel, a cobalt-chromium alloy, a cobalt-molybdenum alloy, a titanium alloy, or a gold alloy.

3. Endoprosthesis according to claim 1 or 2, characterised by the fact that the meshes of the metal wire structure have a pore diameter in the range of from 0.2 to 1.5 mm, and the wires have a diameter of from 0.03 to 1.0 mm.

4. Endoprosthesis according to any one of claims 1 to 3, characterised by the fact that the metal wire structure has inherent osteotropic bioactive properties or is coated with calcium salts, in particular hydroxyl apatite, or with a ceramic material.

5. Endoprosthesis according to claim 1 as an artificial intervertebral disk, comprising a sintered metal wire structure (35) around a plastics disk (34).

6. Endoprosthesis according to any one of claims 1 to 5, characterised by the fact that the metal wire structure comprises meshes having different pore sizes.

7. Process for the manufacture of an endoprosthesis according to claim 1, characterised by the fact that metal wires are transformed on a wire knitting machine capable of producing a knitting of the "rete milanese" kind, into a hollow mesh knitting, and that this hollow mesh knitting is then applied to the metallic endoprosthesis and is sintered to its surface, or is brought into its final shape and then sintered in an oven under vacuum or in a non-oxidising atmosphere, preferably in an argon atmosphere, at a low temperature in the range of from about 600 to 700 °C, the structure being then partially pressed into the surface of the plastics endoprosthesis under heating.

8. Process according to claim 7, characterised by the fact that the hollow mesh knitting is manufactured in one or more layers, as single parts or as an endless fabric, or in the shape adapted to the final configuration.

9. Process according to claim 7, characterised by the fact that the sintered wire structure is made of an osteotropic bioactive material or is coated with such a material, in particular hydroxyl apatite.

10. Process according to any one of claims 7 to 9 for the manufacture of the coating of the shaft of a hip joint prosthesis, characterised by the fact that the knitting of titanium wires having the shape of a cylindrical hose, is drawn over the upper, trapezoid shaped portion of the metallic shaft and sintered thereupon in a sintering oven, coarser mesh pores being produced in the upper portion and finer mesh pores in the lower portion.

11. Process according to any one of claims 7 to 9 for the coating of a hip joint socket of plastics, characterised by the fact that a cap shaped knitting is fitted to a ceramics mould having the same dimension than the hip joint socket and then sintered, that the knitting thus stiffened is placed on the plastics hip joint socket and pressed into the plastics at the flow temperature of the plastics material in such a manner that the loops of the knitting can penetrate only partially into the surface of the hip joint socket, the finishing process being effected subsequently.

12. Process according to any one of claims 7 to 9 for the manufacture of an artificial intervertebral disk, characterised by the fact that a disk shaped plastics piece having a diameter of, e.g., 30 mm is prepared, and two sections cut from an endless titanium wire knitting, having the same diameter, are placed after sintering on both sides of the disk and are partially pressed into the latter in an oven at the flow temperature of the plastics material.

## Revendications

1. Endoprothèse, comprenant une structure de fils métalliques formée par des fils métalliques élastiques, caractérisée en ce que la structure de fils métalliques consiste en un tricotage à mailles creuses du genre "rete milanese", l'endoprothèse étant ou bien une endoprothèse métallique (3, 5, 27), le tricotage à mailles creuses (1, 14, 30) étant alors fritté sur l'endoprothèse, ou bien une endoprothèse en matière plastique (20, 23, 32, 34), le tricotage à mailles creuses fritté (1, 21, 31, 35) étant alors noyé partiellement dans la surface de la prothèse par fusion.

2. Endoprothèse selon la revendication 1, caractérisée en ce que les fils de la structure de fils métalliques consistent en titane, en acier inoxydable, ou en un alliage de cobalt-chrome, de cobalt-molybdène, de titane ou d'or.

3. Endoprothèse selon la revendication 1 ou 2, caractérisée en ce que les mailles de la structure de fils métalliques présentent un diamètre de pores compris entre 0,2 et 1,5 mm, et que les fils ont un diamètre compris entre 0,03 et 1,0 mm.

4. Endoprothèse selon l'une des revendications 1 à 3, caractérisée en ce que la structure de fils métalliques présente d'elle-même des propriétés bioactives ostéotropes, ou bien elle est revêtue de sels de calcium, en particulier d'hydroxylapatite, ou d'une céramique.

5. Endoprothèse selon la revendication 1 sous forme de disque intervertébral, comprenant une structure de fils métalliques (35) autour d'un disque en matière plastique (34).

6. Endoprothèse selon l'une des revendications 1 à 5, caractérisée en ce que la structure de fils métalliques comporte des mailles ayant des grandeurs de pores différentes.

7. Procédé de fabrication d'une endoprothèse selon la revendication 1, caractérisé en ce que des fils métalliques sont transformés, sur une machine à tricoter les fils métalliques agencée pour produire un tricotage du genre "rete milanese", en un tricotage à mailles creuses, et que ce tricotage est ensuite ou bien placé sur l'endoprothèse métallique et fritté avec la surface de ce dernier, ou bien transformé dans sa forme finale et fritté dans un four sous vide ou dans une atmosphère non oxydante, de préférence une atmosphère d'argon, à une température basse comprise dans une gamme de 600 à 700 °C environ, et puis partiellement pressé en chauffant dans la surface de l'endoprothèse en matière plastique.

8. Procédé selon la revendication 7, caractérisé en ce que le tricotage en mailles creuses est fabriqué en une ou plusieurs couches, en pièces ou en continu, ou bien adapté à la forme prévue.

9. Procédé selon la revendication 7, caractérisé en ce que la structure en fils frittée est faite en un matériau bioactif ostéotrope, ou est revêtue d'un tel matériau, en particulier d'hydroxylapatite.

10. Procédé selon l'une des revendications 7 à 9 pour la fabrication d'un revêtement d'une tige de prothèse d'articulation coxofémorale, caractérisé en ce que le tricotage en fils de titane, présentant la forme d'un manchon cylindrique, est enfilé sur la partie supérieure trapézoïdale de la tige métallique et fritté dessus dans un four à fritter, des pores de maille plus grossiers étant formés dans la partie supérieure et plus fins dans la partie inférieure.

11. Procédé selon l'une des revendications 7 à 9 pour la fabrication d'un revêtement d'une cavité cotyloïde en matière plastique, caractérisé en ce qu'un tricotage sous forme de calotte est monté au-dessus d'un moule en céramique ayant les mêmes dimensions que celles de la cavité cotyloïde et puis fritté, et que le tricotage ainsi renforcé est posé sur la cavité cotyloïde en matière plastique et ensuite pressé dans la matière plastique à la température de fluage de cette dernière de telle sorte que les boucles du tricotage ne puissent entrer que partiellement dans la surface de la cavité cotyloïde, le tout passant alors au finissage.

12. Procédé selon l'une des revendications 7 à 9 pour la fabrication d'un disque intervertébral artificiel, caractérisé en ce qu'une pièce en matière plastique sous forme de disque, ayant un diamètre de 30 mm par exemple, est préparée, et deux sections ayant le même diamètre, découpées d'un tricotage de fils de titane sans fin, sont posées après frittage sur les deux faces du disque et sont pressées partiellement dans ce dernier dans un four à la température de fluage de la matière plastique.
